# EUROPEAN PATENT SPECIFICATION

(11) **EP 1 660 888 B1**
(45) Date of publication and mention of the grant of the patent: **25.02.2009**
(21) Application number: 04774869.4
(22) Date of filing: 11.08.2004
(51) Int. Cl.: G01N 33/574, G01N 33/68, G01N 33/543

(54) **METHOD FOR DETECTING LOW LEVELS OF A FUSION PROTEIN**
VERFAHREN ZUM NACHWEIS GERINGER KONZENTRATIONEN EINES FUSIONSPROTEINS
PROCEDE DE DETECTION DE FAIBLES NIVEAUX DE PROTEINE DE FUSION

(30) Priority: 12.08.2003 EP 03077529
(43) Date of publication of application: 31.05.2006
(73) Proprietor: Erasmus University Medical Center Rotterdam, 3015 GD Rotterdam (NL)
(72) Inventor: STAAL, Frank, Jakob, Theodor, NL-2611 VD Delft (NL); VAN DONGEN, Jacobus, Johannes, Maria, NL-2914 LE Nieuwerkerk aan den IJssel (NL)
(74) Representative: Winckels, Johannes Hubertus F.
(86) International application number: PCT/NL2004/000562
(87) International publication number: WO 2005/015235

(56) References cited:
- WO-A-92/21032
- WO-A-02/054074
- CORDELL J L ET AL: "DETECTION OF NORMAL AND CHIMERIC NUCLEOPHOSMIN IN HUMAN CELLS" BLOOD, W.B.SAUNDERS COMPAGNY, ORLANDO, FL, US, vol. 2, no. 93, 15 January 1999 (1999-01-15), pages 632-642, XP002902335 ISSN: 0006-4971
- RABBITS T.H.; STOCKS M.R.: 'Chromosomal translocation products engender new intracellular therapeutic technologies' NATURE MEDICINE vol. 9, no. 4, April 2003, pages 383 - 386

## Description

The invention relates to the detection of, among others, tumor-specific fusion proteins. More specifically, the invention relates to techniques that indicate the presence of chromosomal translocations by detecting the presence of a fusion protein in a biological sample.

In the diagnosis of various types of cancer, such as leukemias, lymphomas and solid tumours, chromosomal aberrations are frequently used for classification into prognostically relevant subgroups. Many of these chromosomal aberrations result in fusion genes, i.e. aberrantly coupled genes coupled via the upstream part of one gene to the downstream part of the other gene, or vice versa. Fusion genes can be transcribed into fusion gene transcripts and translated into fusion proteins. Generally, fusion proteins play an important role in the oncogenetic process. For example, approximately 35% of adult patients with acute lymphoblastic leukemia (ALL) and approximately 95% of patients with chronic myeloid leukemia (CML) have a specific chromosomal defect in their leukemic cells, i.e. a translocation between chromosomes 9 and 22 that creates the Philadelphia (Ph) chromosome. This translocation occurs at the site in the genome of a protein tyrosine kinase named ABL, creating the abnormal BCR-ABL fusion protein, a gene product of the in-frame fusion of the *ABL* gene with another gene called *BCR*. Generally, fusion proteins play an important role in the oncogenetic process. For example, the kinase activity of ABL in the BCR-ABL fusion protein is activated and deregulated, driving the uncontrolled cell growth observed in ALL and in CML.

When ALL is diagnosed in a patient, the total number of leukemia cells typically lies within the range of 10¹¹ to 10¹². Cytostatic or cytotoxic treatment induces complete remission in the majority of patients with lymphoid malignancies. Nevertheless, many of these patients relapse. Complete remission does not mean that the leukemic cells are totally eradicated from the body but that their level is beyond the sensitivity level of classical cytomorphologic methods. Since the detection limit of cytomorphological techniques is not lower than 1-5% malignant cells, this means that up to 10¹⁰ malignant cells can still remain in the patient, also indicated as the "minimal residual disease" (MRD). Apparently the current treatment protocols are not capable of killing all malignant cells in these relapsing patients, although they reached so-called complete remission according to cytomorphological criteria. Thus, it is obvious that cytomorphological techniques can only provide superficial information about the effectiveness of treatment.

Detection of low numbers of residual malignant cells during early treatment response allows a precise assessment of the kinetics of tumour decrease during chemotherapy. It is now established that MRD information represents a powerful prognostic factor for final outcome. Detection of an increase of the MRD level enables to anticipate impending relapse. Techniques with a high sensitivity to detect MRD are therefore crucial to obtain a better insight in the reduction of tumour mass during induction treatment and further eradication of the malignant cells during maintenance treatment.

Existing techniques for detecting chromosomal aberrations include traditional techniques such as cytogenetic analyses and biomolecular technology, such as Southern blotting or Fluorescent *in situ* hybridisation (FISH) analysis. However, the detection sensitivity of cytogenetics, FISH analysis and Southern blotting is typically not lower than 1-5%, which makes them unsuitable for the detection of MRD.
PCR, although in essence well-suited for rapid and massive diagnostic testing or even screening, allows only 0.1 to 4-5 kb of nucleic acid (DNA or RNA) to be analysed routinely per PCR analysis, which greatly hampers rapid screening of vast stretches of chromosomes and breakpoint clusters or fusion regions within the chromosomes or their gene-products. An additional disadvantage of PCR is its inherent sensitivity to mismatched primers. Small alterations which can always be present in the nucleic acid sequence of the gene fragment complementary to the primer will make it impossible to operate the PCR with the wanted effect and may result in misdiagnosis and false-negative results. Especially false-negative results render a PCR-based diagnostic test, albeit very specific, unsuitable for reliable diagnosis, and it goes without saying that only a reliable diagnosis of malignancies can contribute to an insight into the prognosis and to the design of an adequate therapy.
As indicated above, the majority of currently available techniques aim at detecting specific chromosomal aberrations involve analysis at the chromosomal or nucleic acid (DNA or RNA) level. It is however also possible to detect a chromosomal aberration at the protein level.

Considerable effort has been undertaken in the field of leukaemia diagnostics to develop a suitable technology for the immunological detection of a fusion protein resulting from a chromosomal aberration. Many of those studies focussed on finding immunological reagents (antibodies) that are exclusively reactive with tumour-specific proteins and which do not at the same time give immunological detection of non-fusion proteins that are normally also produced by the body (see for example Nagasaki et al., J.Imm. Methods 162, 235-245, 1993 and Van Denderen et al., J Exp Med. 1989 169(1):87-98). Usually, such antibodies cross-react with normal cellular proteins. Only when specific fusion points are known, it may be possible to select specific immunological reagents that react exclusively with the tumour-specific protein, by selective binding to the fusion point epitope. However, the variation in fusion points is so large between patients (different breakpoints at the DNA level) and within patients (mRNA splice variants) that specific immunological detection only works in a few occasions, often solely on a patient-by-patient basis (see Van Dongen et al. Leukemia 1999; 13:1901-1928). Furthermore, diagnostic tests involving immunodetection of the fusion point of a fusion protein have the great inherent disadvantage that they require specialised and well-equipped laboratories and trained and highly skilled personal. Also, the above tests are typically only used in suspected cases of malignancies, and are not suitable for large scale screening of leukaemia patients for the presence of chromosomal aberrations.

The development of a rapid and more convenient immunological method allowing the exclusive detection of a fusion protein A-B was a major step forward in the area of diagnostic tests aimed at detecting a fusion protein (see PCT/NL98/00289). The reported method involves the application of a so-called catching antibody which recognizes one part of a fusion protein (part A) and a labelled detection antibody which recognizes an other part of a fusion protein (part B). In such a system, a catching antibody is bound to a solid support layer, such as an ELISA plate or a dipstick (P. Berendes, "Recognition of tumor-specific proteins in human cancer. PhD Thesis Erasmus University Rotterdam, ISBN 90-73436-36-2). A catching antibody can also be immobilized onto beads that can be analysed by flow cytometry (see for example PCT/NL01/00945). Following incubation of a bead-bound catching antibody with a cellular lysate suspected of containing a fusion protein, bound fusion protein is detected by a labelled detection antibody.

A (bead-based) catching/detection antibody system permits the exclusive detection of a fusion protein and is elegant and easy to perform, especially when using-flow cytometry. Such a detection system also allows high-throughput diagnosis of many clinical samples. At the time of diagnosis of a malignancy involving a fusion protein, a diagnostic sample typically contains a relatively high number of malignant cells with a fusion protein compared to the number of normal cells which might contain native (non-fused) proteins. When ALL is diagnosed in a patient, the total number of leukemia cells is approximated to 10¹¹ to 10¹², of which approximately generally 25% to 98% contain a fusion gene encoding a fusion protein. A catching/detection antibody system is therefore suitable for the rapid screening for chromosomal aberrations which give rise to a fusion protein. Unfortunately however, the sensitivity of a catching/detection antibody system often appears insufficient in detecting low frequencies (<1%) of malignant cells in a large background of normal cells. As an example, current catching/detection antibody systems are in general not adequate in detecting MRD during treatment follow-up. The diagnostic application of a catching/detection system is therefore essentially limited to the stage of initial diagnosis, when the relative frequency of malignant cells in bone marrow or blood is high (generally >10%). Taken together, there is a specific need for a method that allows to exclusively and conveniently detect a fusion protein in a sample, even if the sample contains only minimal amounts of the fusion protein.

It has now been recognized that, in current methods, a fusion protein and a non-fused native (i.e. wild-type) protein typically compete for specific binding to a probe that is used for the detection of the fusion protein. For example, a fusion protein A-B and a non-fused native protein A will compete for binding to a catching antibody, or other type of probe, specifically reactive with part A of the fusion protein. Likewise, a fusion protein A-B and a non-fused native protein B compete for specific binding to a catching antibody that is reactive with part B of the fusion protein. Thus, the sensitivity of a catching/detection antibody system for detecting a fusion protein comprising parts of at least two native proteins is largely determined by the proportion of a fusion protein present in a sample relative to said native non-fused proteins. When a native protein is in large excess of a fusion protein of interest, the native protein is like to occupy most, if not all, binding sites of a catching antibody, thereby essentially preventing binding of a fusion protein. Furthermore, even if some fusion protein is efficiently caught by a catching antibody, the signal intensity indicative of binding of a labelled detection antibody may not be sufficient to yield a reliable test result.
The invention provides the insight that the sensitivity of a method for detecting a fusion protein in a sample can be increased by depleting said sample of one or more native proteins prior to detecting the presence of a fusion protein, to enrich said sample for said fusion protein relative to one or more 'competing' native proteins. Provided is a method to detect a fusion protein (e.g. resulting from a chromosomal translocation) in a sample via the detection of a fusion protein comprising an amino-terminal fragment and a carboxy-terminal fragment which are each corresponding to a different native protein, said method comprising (i) contacting said sample with at least one binding molecule specifically reactive with a part of the native protein that is not present in the fusion protein, under conditions that allow the formation of a complex between said at least one binding molecule and said native protein; (ii) removing said complex from said sample to essentially deplete said sample of said native protein but not the fusion protein and (iii) detecting said fusion protein in said sample using at least one antibody directed against said fusion protein. The term native protein or relevant native protein as used herein refers to a non-fused, wild-type protein which can compete with a fusion protein of interest for binding to a probe or reagent that is used for the detection of a fusion protein, such as for instance a catching antibody. A native protein of the invention can adopt a native conformation (the three dimensional structure of a protein when in a physiological environment) or a denatured conformation.
According to the invention, a fusion protein is derived from a fusion gene of a malignant cell or from an artificially produced fusion gene. In a fusion protein comprising an amino-terminal fragment and a carboxy-terminal fragment which are each corresponding to a native protein, said fragments are typically directly fused or connected to each other via a breakpoint fusion or fusion region such that these fragments together represent the whole fusion protein. The length or the size of a fragment, whether amino-terminal or carboxy-terminal, can vary. If the fusion region is located approximately halfway a fusion protein, the fusion protein comprises an amino (N)-terminal fragment and a carboxy (C) - terminal fragment of approximately the same size. In other cases, the fusion region is located more closely towards either the C-terminal or N-terminal end of the fusion protein, such that the fusion protein consists of a large N-terminal fragment and a small C-terminal fragment or a large C-terminal fragment and a small N-terminal fragment, respectively. A fusion protein of the invention contains sufficient amino acids upstream and downstream from the fusion region to allow for (epitope) recognition by an antibody probe specifically reactive with parts of the fusion protein located at opposite sides of the fusion region or a binding fragment functionally equivalent thereto.

In a method of the invention, a sample is enriched for a fusion protein relative to one or more 'competing' native proteins, resulting in increased binding of a fusion protein to a probe directed against said fusion protein. A method provided is particularly suitable to increase the sensitivity of a catching/detection antibody or sandwich system for the detection of a fusion protein. Thus, the invention provides a solution to a major problem encountered using existing detection methods wherein a native protein saturates or "consumes" a probe that is used to detect a fusion protein, such as a catching antibody, thereby preventing the binding of a fusion protein to said probe. Obviously, probe saturation with native protein reduces the sensitivity of detecting a fusion protein present in the same sample. By depleting a sample of a competing native protein according to the invention, the ratio fusion protein/native protein is strongly increased, thus favouring binding of the detection probe, and detection of the fusion protein. With a method provided herein, it is now possible to specifically detect the presence of a fusion protein in relatively rapid and simple fashion, even if said fusion protein is present in a sample in only a limited amount. For example, it allows to apply a catching/detection antibody system to detect a tumour-specific fusion protein in a sample comprising only a small population of fusion protein-positive malignant cells in a large background of normal cells, which contain a large number of competing, native proteins.

Furthermore, with a method according to the invention it is now also possible to calculate the amount of native protein (as a measure for the amount of normal cells) relative to the amount of fusion protein (as a measure of the amount of malignant cells. The obtained ration of normal protein versus fusion protein can be used to determine the relative frequency of malignant cells, i.e. the level of MRD.

The invention provides the insight that the sensitivity of detecting the presence of a fusion protein in a cell, e.g. a chimeric oncoprotein, in a malignant cell, can be increased by depleting such a cell of at least one relevant native protein. Of course, it is possible to deplete more than one native protein prior to detection of a fusion protein. Needless to say, use of a method provided herein is most beneficial when detecting a fusion protein in a sample that also contains a significant amount of native proteins which can compete in binding to a probe that is used to catch a fusion protein. In one example, a method is provided for the detection of MRD by determining the presence of malignant cells in a sample, said method comprising detection of a tumour-specific fusion protein comprising parts of at least two native, non-tumour specific proteins in a sample, said detection step being preceded by a "preclear" step wherein said sample is depleted of at least one of said native proteins, which would otherwise compete with the detection of the fusion protein.

In a method of the invention at least one binding molecule is used to remove one or more native proteins from a sample. A sample comprises a biological sample such as a blood sample, serum sample, tissue sample, bone marrow, cerebrospinal fluid sample, biopsies and other samples that may contain one or more cells expressing a fusion protein encoded by a fusion gene. A sample is treated in such a manner that a binding molecule has sufficient access to a native protein. Because many cellular proteins are localized intracellularly, this is typically achieved by disrupting the membrane integrity of a cell. For example, a sample is treated in such a manner that it yields a cell lysate or a cell homogenate, for instance obtained by mechanical rupture of cells or by exposing cells to a cell lysis buffer containing a detergent. A suitable binding molecule preferably comprises a protein or a polypeptide, like an antibody or an antibody fragment. However, other types of binding molecules are also instrumental in practicing a method provided. A specific antibody or fragment thereof can be obtained using various standard procedures known in the art, including methods to raise polyclonal antibodies in a laboratory animal, methods to obtain monoclonal antibodies using conventional hydridoma technology, and single-chain Fv antibody fragments (scFvs) using a phage display selection process. Such an antibody or antibody fragment is herein further referred to as "depleting-antibody".

For the design or development of a binding molecule that is reactive with a native protein but not with a fusion protein comprising a part of said native protein (such as a depleting antibody), it is of course crucial to know which part or fragment of said native protein is part of the fusion protein and which part is not. In other words, it is preferred that the fusion point or fusion region of a fusion protein to be detected is established. A method of the invention is advantageously used to detect a fusion protein that is the result of a known chromosomal aberration. In these cases, the resulting fusion genes and fusion proteins have often been characterized into detail. Well known examples are the translocations resulting in *BCR-A.BL* fusion genes found in >95% of cases of CML and in 30% of cases of adult ALL and the *TEL-AML1* fusion genes which are found in 25-30% of childhood ALL cases. However, many more fusion genes and encoded fusion proteins are known to be involved in leukemia, such as E2A-PBX1, ETO-AML1 and PML-RARα.

It is to be understood that application of the present invention is not limited to the detection of leukemia; several other, non-leukemic diseases are characterized by the occurrence of fusion genes and fusion proteins. For example, the following ALK fusion genes can be observed in anaplastic large cell lymphoma (ALCL): the fusion gene *NPM-ALK* resulting from chromosome aberration t(2;5)(p23;q35); fusion gene *TPM3-ALK* resulting from chromosome aberration t(1;2)(q25;p23); fusion gene *TFG-ALK* resulting from t(2;3)(p23;q21) and fusion gene *ATIC-ALK* from inv(2)(p23q35). Depletion of native ALK protein from a sample according to the invention using a depleting antibody reactive with native ALK but not with the fusion protein will increase the sensitivity of a subsequent catching/detection assay. In another embodiment, the invention is advantageously applied to detect a chromosomal aberration observed Ewing's sarcoma (EWS), such as t(11;22)(q24;q12) resulting in the EWS-FLI1 fusion protein, t(21;22)(q22;q12) resulting in the EWS-ERG fusion protein and t(7;22)(p22;q12) giving rise to the EWS-ETV1 fusion protein. Ewing's sarcoma (EWS) / Peripheral Primitive Neuroectodermal Tumours (PNET) of bone is a type of cancer usually found in children and young adults. The peak incidence is between ages 10 and 20, it is less common in children under 5 or in adults over 30. Ewing's sarcoma can occur in any bone in the body; the most common sites are the pelvis, thigh, lower leg, upper arm, and rib. Depletion of normal cellular EWS protein (and/or optionally depletion of the fusion partner gene such as FLI1, ERG or ETV1) in a preclear step according to the invention will increase the sensitivity of detecting a fusion protein related to Ewing's sarcoma.

Thus, the invention allows a person skilled in the art to apply and perform the invention without undue burden to the diagnosis or classification of any type of disease that is associated with the occurrence of a fusion gene that is translated into a fusion protein comprising an amino-terminal fragment -and a carboxy-terminal fragment which are each corresponding to a native protein.

Based on the structure of a fusion protein, one can easily determine which part of the native protein is not present in the fusion protein. A binding molecule specifically reactive with this part, or a (polypeptide) stretch thereof, can be used to essentially remove the native protein, but not the fusion protein, from a sample. A person skilled in the art will recognize which steps to follow to obtain a binding molecule specifically reactive with the native (wild-type) protein but not with the fusion protein. In a preferred embodiment, such a specific binding molecule is an antibody or a binding fragment functionally equivalent thereto.

Methods of producing an antibody are known to those skilled in the art. For example, to obtain a polyclonal antibody, a laboratory animal is immunized with an immunogen. In a method of the invention, said immunogen preferably is a recombinant protein fragment or a synthetic peptide corresponding to the selected stretch or domain of the native protein. The animal's immune response is monitored by taking test bleeds and determining the titer of the reactivity. When appropriately high titers are obtained, blood is collected from the animal and antisera are prepared. Further fractionation of the antisera to enrich for antibodies reactive with the native protein can be done if desired. See e.g. Harlow et al. Antibodies. A Laboratory Manual, Cold Spring Harbor Publications, New York (1988). Monoclonal antibodies can be obtained by various techniques known in the art, for example by fusing spleen cells of immunized mice with a myeloma cell line by the addition of polyethylene glycol (PEG). Fused cells are cultured in a selection medium, e.g. medium containing a mixture of hypoxanthine, aminopterin and thymidine. Fused cells which survive in this selection medium are tested for the production of the desired antibody (for instance by a solid-phase immunoassay such as ELISA) and, if positive, the cultures are cloned so that there is only one cell in each culture well. This produces a clone of cells from a single progenitor which is both immortal and a producer of monoclonal antibody. Antibodies obtained can be characterised using conventional immunodiagnostic techniques e.g. by Western blotting using lysates of cells expressing either the (recombinant) native protein or the (recombinant) fusion protein. Only those antibodies reactive with the native protein A but not with the fusion protein A-B are suitable for use as depleting antibody in a method according to the invention.

According to the invention, a complex between a depleting antibody and a native protein is removed from a sample prior to detecting a fusion protein. In one embodiment, a method provided entails rapid and selective removal or precipitation of a native protein out of a cell lysate or homogenate, via for example the formation of an antibody/antigen complex. Hereto, a sample is contacted with at least one binding molecule specifically reactive with a native protein under conditions that allow the formation of a complex between said binding molecule and said native protein. Various separation techniques can be used in a method according to the invention to remove a complex from a sample. Commonly used methods to precipitate an antibody/antigen complex out of a crude mixture include solid phase immunoprecipitation. In one embodiment, said complex is removed from said sample via the addition of solid beads or a microspheres capable of binding said complex, to form a suspension of beads in an essentially liquid sample. According to the invention, a suitable bead comprises a Sepharose bead, or a polystyrene bead, or any kind of solid particle that allows the simple removal of a bead-bound complex from a sample. The advantage of solid beads is that they can be simply pelleted from a sample, typically by centrifugation of the suspension. Alternatively, a precleared sample is obtained by filtration of the suspension. In one embodiment of the invention, a sample is contacted with a depleting antibody to form a complex with a native protein and an immunoglobulin binding protein immobilized onto a bead is used to remove-said complex from said sample. Various types of immunoglobulin binding proteins exist which can suitably be used (Antibodies, a Laboratory Manual, E. Harlow & D. Lane, (1988)). These include protein A, protein G and bacterial cell ghosts of *Staphylococcus aureus. S. aureus* has a special protein on its surface called Protein A which is a versatile, broad range IgG-binding reagent. Protein A binds the Fc portion of antibodies (IgG class) without disturbing their binding of antigen. Antibodies from many mammalian species will generally react with Protein A. In another embodiment, GammaBind G Type 2 or Protein G is used to remove a complex of a depletion antibody and a native protein from a sample. GammaBind™ G Type 2 and Protein G are recombinant engineered forms of streptococcal protein G. Both proteins can be produced in *Escherichia coli.* They bind to the constant region of many types of immunoglobulin G, and are widely used to detect, quantify and purify IgG antibodies and antigen/antibody complexes. GammaBind G Type 2 and Protein G are the most universally applicable antibody binding proteins available. Compared with Protein A, they bind tightly and specifically to antibodies from many different species. For example, GammaBind G Type 2 binds better to mouse and rat IgG, GammaBind G Type 2 and Protein G bind selectively to all subclasses from human IgG. Bacterial ghosts, immunoglobulin binding proteins and beads coated with immunoglobulin binding proteins can be commercially obtained from various suppliers, such as Pharmacia Biotech AB, Sigma Aldrich or Pierce. In a method provided herein, following the formation of a complex between a native protein and a depleting antibody, bacterial ghosts or coated beads are mixed with the cell sample or cell homogenate, during which time the immunoglobulin binding protein attaches to the depleting antibody. A brief and simple centrifugation or filtration step can separate the bacterial ghosts or beads with attached depleting antibody / native protein complex from the sample.

In a preferred embodiment however, a binding molecule (preferably a depleting antibody) is directly coupled to or immobilized onto a bead or another solid particle. Display of biochemical reagents on synthetic microbeads is a prevailing trend in the development of bioanalytical assays. Commercial sources (e.g. Spherotech, IL; Bangs, IN; Polysciences; PA); Molecular Probes, OR) for bead-based display systems (e.g., covalent coupling, biotin-streptavidin, His-tag) are currently available. Use of a depleting antibody which is directly conjugated to a bead does not require the intermittent use of an immunoglobulin binding protein and will in general result in increased specificity. Following incubation for a sufficient time period, beads with attached depleting antibody complexed to native protein can be removed from a sample, such as a lysate or homogenate. Removal of beads from a sample is easily and rapidly achieved by for instance centrifugation, resulting in a sample that is essentially depleted of a native protein which would otherwise compete with a fusion protein for binding to a probe directed against said fusion protein. Because this depletion step results in a sample that is enriched for a fusion protein relative to the "competing" native, non-fused protein, the probability that a fusion protein will bind to such a probe, e.g. a catching antibody, is significantly increased. For example, a method provided now allows to detect a fusion protein using a catching/detection system with increased sensitivity.

In another embodiment of the invention, a depletion antibody or any other type of suitable binding molecule, is conjugated to a magnetic bead. This allows the removal of a bead-bound complex from a sample using immunomagnetic separation (IMS). IMS is a technique that involves adding magnetic beads coated with a binding molecule, which is specifically reactive with an antigen of interest, to a cellular lysate. The beads and lysate are mixed, during which time a complex is formed between a depleting antibody or other binding molecule and a native protein to form a (antigen/antibody) complex. After a certain incubation period, a magnetic field is applied to separate the magnetic beads or magnetic particles with attached complex from other substances, leaving behind a depleted or 'pre-cleared' sample. Suitable magnetic beads are for example those marketed by Spherotec Inc. (www.spherotech.com) under the brandname SPHERO magnetic particles. They are prepared by coating a layer of magnetite and polystyrene onto monodispersed (i.e.. uniform sized) polystyrene core particles (see US Patent No. 5,091,206). As a result, the particles are spherical in shape, and paramagnetic in nature. They are also very uniform in size. The magnetite contents of these magnetic particles can be adjusted but in general it represents about 10% to 15%. The magnetic particles can be easily separated from a suspension magnetically. These particles become non-magnetic when removed from a magnet, and do not retain any detectable magnetism even after repeated exposure to strong magnetic field. The magnetic particles can be used for cell separation, affinity purification, DNA probe assays, magnetic particle EIA, etc. Various sizes of beads, be it Sepharose beads, polystyrene beads, latex beads, magnetic beads or the like, are commercially available. In a preferred embodiment, beads with a relatively small diameter are used, because for a certain volume of beads, small beads have a larger surface area compared to large beads. The larger the bead surface, the more binding molecules can be immobilized onto a bead and the more efficient a native protein can be removed from a sample per volume of beads. This is especially advantageous when removing a native protein from a sample with a small sample volume.

As said, the invention solves the problem that a catching/detection antibody system, although rapid and easy to perform, is often not sensitive enough to detect the presence of low amounts of a fusion protein in a sample. A method is provided to detect a fusion protein comprising parts of two different native proteins with increased sensitivity by including a pre-treatment step to obtain a sample that is enriched in a fusion protein of interest relative to one or both of the native proteins. This enriched, or precleared, sample, is processed further to detect the presence of a fusion protein. In theory, a fusion protein A-B present in a sample that is completely depleted from native protein A using a method provided, can be detected using a single antibody (or functional equivalent thereof) directed against part A of the A-B fusion protein. However, as it is very difficult in practice to obtain a precleared sample which does not contain any native protein A, use of a single A-specific antibody may not always give the desired detection specificity. Thus, when aiming for the exclusive detection of an A-B fusion protein, it is preferred to use at least one additional antibody that is directed against part B of the fusion protein. For example, a fusion protein is detected using a set of at least a first and a second antibody, each antibody capable of recognizing a binding site positioned at opposite sides of the fusion region of said fusion protein. In a preferred embodiment of the invention, the invention provides a method wherein a fusion protein derived from a fusion gene is detected by flow cytometric detection or by any other convenient way to measure bead-associated fluorescence using at least two antibody probes, whereby at least one is directed against a protein fragment comprising the amino-terminal fragment of the fusion protein, and at least one other one is directed against a protein fragment comprising the carboxy-terminal fragment of the fusion protein.

The use of microspheres, beads, or other particles as solid supports for antigen-antibody reactions in order to detect antigens or antibodies in a sample is particularly attractive when linked to flow cytometry (see e.g. US 6,159,748). In a preferred embodiment, an antibody probe is coupled to a bead that allows the detection of a fusion protein via flow cytometry. Flow cytometers have the capacity to detect particle size differences and are highly sensitive fluorescence detectors. Microspheres can be sized by forward angle light scatter (FALS) or electronic volume. Used in conjunction with right angle light scatter (RALS), a flow cytometer can distinguish between single and aggregated particles.

A method according to the invention is very attractive for the detection of a tumour specific protein comprising an amino-terminal fragment and a carboxy-terminal fragment which are each corresponding to a different non-tumour-specific protein. Said tumour-specific fusion protein is for example a result of a Philadelphia chromosome aberration, creating the abnormal BCR-ABL protein. Prior to detecting, one or both of the native proteins BCR and ABL are depleted from a sample to be analysed by using at least one binding molecule specifically reactive with (a fragment of) the ABL or BCR protein, but not with the BCR-ABL fusion protein. Following removal of the complex comprising BCR and/or ABL, the BCR-ABL fusion protein is detected, preferably using a set of at least a first and a second antibody, whereby each antibody recognizes a binding site positioned at opposite sides of the fusion region of the fusion protein. The fusion protein can be detected using a catching/detection system, for instance using flow cytometry, ELISA, or a dipstick method (see also the Example below).
A method of the invention is especially suitable to detect very low numbers of cells expressing a fusion protein (<1% such as encountered in MRD). However, it is of course also advantageously used to increase the sensitivity of detection methods which do not require such low detection limits, such as those used for the initial diagnosis of a disease that is correlated with the presence of a fusion protein. Also in these cases, removal of one or more native proteins prior to detecting a fusion protein will result in enhanced sensitivity. At the stage of diagnosis, it is often desired to investigate the occurrence of different fusion gene proteins, preferably simultaneously in one tube. This is not because a particular malignancy will have more than one fusion gene protein, but because it is convenient to have a single test tube for detection of several well-established fusion gene proteins within one disease category *e.g*., acute myeloid leukemia (AML) or precursor-B-ALL (Table 1).

**Table 1. Examples of combined detection of several fusion proteins in a single tube.**

| **Disease** | **Chromosomal** | **Fusion protein** | **Relative frequency** | |
|---|---|---|---|---|
| **category** | **aberration** | | | |
| | | | **children** | **adults** |
| AML | t(8;21) (q22;q22) | AML1-ETO | 10%-14% | 6%-8% |
| | t(15;17) (q22;q21) | PML-RARA | 8%-10% | 5%-15% |
| | inv(16) (p13;q22) | CBFB-MYH11 | 5%-7% | 5%-6% |
| | | | | |
| Precursor B-ALL | t(1;19) (q23;p13) | E2A-PBX1 | 5%-8% | 3%-4% |
| | t(4;11) (q21;q23) | MLL-AF4 | 3%-5% | 3%-4% |
| | t(9;22) (q34;q11) | BCR-ABL | 3%-6% | 25%-40% |
| | t(12;21) (p13;q22) | TEL-AML1 | 25%-30% | 0%-2% |

In a preferred embodiment, a method provided herein comprises the flow cytometric detection of different types of fusion proteins, preferably in a single tube assay, by using different bead-bound catching antibodies against one part of the different fusion proteins and the relevant corresponding detection antibodies against the other part of the fusion proteins. By combining FALS and fluorescence, it is practical to use beads of several different sizes, each bead coated with a different catching antibody, for the simultaneous detection of multiple fusion proteins. Microspheres can be coated with proteins passively or covalently depending on their chemical makeup. Based on different flow cytometric characteristics of the beads (e.g. size, fluorochrome colour, intensity of fluorochrome staining, or side scatter characteristics), multiple fusion proteins can be specifically detected in the same assay. This also includes the detection of fusion proteins from various variant translocations of the same target gene as well as fusion proteins from translocations with variant breakpoints. An essential part of a method according to the invention for the detection of a one or more fusion proteins, be it using a catching/detection system or any other kind of detection procedure, lies in the fact that fusion protein detection is preceded by the depletion of one or more relevant native, non-fused proteins. This can be achieved using one or more binding molecules, such as depletion antibodies, that can specifically recognize and bind to a native protein to form a complex. Different binding proteins may be attached to different single solid support surfaces, like a bead. Alternatively, multiple binding partners specifically reactive with different native proteins are coupled to the same bead such that only a single bead can be used to simultaneously deplete a sample of multiple native proteins. This is especially of interest when a method according to the invention is used to investigate the occurrence of different fusion proteins simultaneously in one tube, e.g. for the diagnosis of a malignancy within one disease category, particularly when using patient samples with a low tumour load (e.g. less than 5%) at diagnosis.
Thus, a method of the invention allows for the simultaneous detection of a first fusion protein A-B, comprising the N-terminal fragment of the native protein A and the C-terminal fragment of the native protein B, and a second fusion protein C-D, comprising the N-terminal fragment of the native protein C and the C-terminal fragment of the native protein D, in a single tube assay with a high specificity and improved sensitivity compared to existing detection methods. In this specific example, a patient sample with a low tumour load is for example contacted with a depletion antibody specifically reactive with the C-terminal fragment of protein A (anti-A) and with a depletion antibody specifically reactive with N-terminal fragment of protein D (anti-D), to deplete the sample from native proteins A and D, respectively. The anti-A and anti-D depletion antibodies are preferably immobilized onto a (small) bead, even more preferred onto the same bead for reasons of efficiency and ease of use. A simple centrifugation step is in general sufficient to separate the complex, in this specific example consisting of beads/anti-A/protein A/anti-D/protein D, from the sample. As a result, a sample is obtained which is essentially depleted of native proteins A and D. This will improve the likelihood that the fusion proteins A-B and C-D will be recognized by their specific probes, e.g. a catching antibody specifically reactive with the N-terminal fragment of fusion protein A-B and the C-terminal fragment of fusion protein C-D.

In addition to the limited sensitivity, current methods for detecting a fusion protein in a sample face the common problem that the possibility to quantify the amount of fusion protein detected are limited. Especially when applying these methods as diagnostic tools, it is highly desirable to quantify, or at least semi-quantify, a fusion protein. If possible at all, a detection signal, such as fluorescence intensity, which indicates the presence of a fusion protein can be expressed relative to an internal control factor assumed to be constant irrespective of whether or not a fusion protein is present. Such a factor can for instance be the number of cells analysed or the total protein content of a sample. This relative value can be used for the purpose of comparison between different samples, e.g. a positive and a negative control sample. Obviously, it would be far more informative to directly relate the presence of a fusion protein comprising parts of two native proteins to one, or even both, non-fused native protein(s).
Thus far, none of the existing detection methods allows for such a quantification. Importantly, the present invention now also provides a simple and straightforward solution to this problem. A complex containing one or more depleted native protein(s) that is removed from a sample in a method of the invention is preferably analysed further to indicate the amount of native protein(s) depleted from said sample. In a preferred embodiment, this analysis comprises the use of at least one reagent capable of specifically binding to one or more components of the depleted or isolated complex, followed by determining the binding of said reagent to said complex as an indication of the amount of native protein that was depleted from said sample. A reagent preferably comprises a binding partner, like an antibody or a functionally equivalent fragment thereof, capable of binding a depleted native protein. In a preferred embodiment, a depleted complex is detected using a reagent directed against a depleted native protein, at a region which is distinct from the site of interaction between the native protein and a depletion antibody. More preferred, a reagent for detecting a depleted native protein reacts with that part of the native protein which is also present in the fusion protein. For example, a depleted protein A is advantageously detected using the "'catching antibody" used for the detection of the A-B fusion protein as a reagent.
A reagent is preferably provided with a reporter molecule or label, like a fluorochrome, allowing detection of a complex via the binding of a labeled-reagent to a depleted protein.
Detection of a complex to determine the amount of native protein depleted form a sample is advantageously carried out using flow cytometry. In one embodiment, a depletion antibody is conjugated to a bead. Suitable beads comprise those discussed above for detection of a fusion protein, including magnetic beads compatible with flow cytometry.
When the amount of depleted native protein is known, this information can be used as an indication of the amount of cell material or total protein content. In other words, the depleted protein can serve as a kind of "internal control" in the diagnostic test. When relating the presence (expression) of a protein in an isolated complex to the amount of cell material or to the number of cells it is of course preferred that the isolated complex-contains a sufficient amount of said native protein to be detected by a reagent. Thus, a native protein to be depleted and analysed further as measure of the amount of cell material preferably comprises a protein that is relatively ubiquitous in said cells. In addition, it is important that the expression of said native protein is not subject to large variations, e.g. between individual cells or under different cellular conditions. In a preferred embodiment, said native protein to be depleted and analysed further is an abundant protein with a stable expression, such as a housekeeping protein. For example, when detecting the BCR-ABL fusion protein in a sample it is advantageous to deplete and subsequently detect the native ABL protein because ABL is an abundant protein.
However, a fusion protein does not always comprise a fragment of an abundant native protein. For example, in the E2A-PBX1 fusion protein the N-terminal transactivation domain of the basic helix-loop-helix (bHLH) transcription factor, E2A, is joined to the majority of the pre-B-cell leukemia transcription factor 1(PBX1). Transcription factors are typically expressed at a low level and in a cell-type specific fashion. Thus, in these cases it is not preferred to use any of the depleted native proteins as an internal control. Instead, it is advantageous to use an additional (antibody) probe which simultaneously depletes a sample of an abundant protein. To illustrate this further, in the situation depicted above, a sample e.g. a cell lysate is contacted with a bead that is coated with two antibodies: one antibody reactive with native E2A but not with the E2A-PBX1 fusion protein to deplete the sample of competing E2A protein, and one antibody reactive with a housekeeping protein (such as ABL) to deplete the sample at the same time of a housekeeping protein that can serve as internal control.
According to the invention, (semi)-quantification of a depleted native protein can be performed by constructing a standard curve relating a measured quantity (e.g. fluorescence) to "known" amounts of native protein. Preferably, "known" samples contain the native protein in amounts chosen to span the concentration range of native proteins that are expected in the "unknown" samples (e.g. using (serial) dilutions of normal cells). Such a standard curve can then be used to determine concentrations of the depleted native protein substance in an "unknown" sample (or a dilution thereof) containing the depleted complex.

The invention provides a method as discussed above using probes that can be labelled or conjugated with reporter molecules, such as biotin, digyoxigenin, enzymes such as peroxidase, alkaline phosphatase, or other reporter molecules or reporter particles, such as beads, known in the art. The invention further provides a diagnostic kit comprising all the means, such as binding molecules, (conjugated) beads, (labelled) probes or reagents or substrate or instructions, necessary to carry out the method according to the invention. Methods or diagnostic kits provided by the invention are preferably used to detect chromosomal aberrations found with certain types of cancer, for example with leukaemia, be it in the detection of (residual) cancer in patients or the screening for cancer in larger populations as a whole.

Strengths of a method according to the invention are: 1) the ability to simultaneously, but discretely, analyse multiple fusion proteins (most relevant at the time of diagnosis); 2) the simplicity of binding proteins to microspheres; 3) the ability of flow cytometry to detect small particle size differences; and 4) the exquisite sensitivity of flow cytometry as a detector of different wavelengths of fluorescence, simultaneously. Available auto-sampling systems make it even more appealing in this regard.
A method of the invention is advantageously used in diagnostic testing of biological samples such as blood samples, serum samples, samples of cells, tissue samples, cerebrospinal fluid, bone marrow, biopsies, for chromosomal aberrations. The invention provides a method to be used in diagnostic testing where both a high sensitivity as well as a high specificity is required. A method provided now allows to both diagnose a malignancy and closely monitor MRD during follow-up of patients with various types of cancer which involve chromosomal translocations, inversions or deletions that give rise to a fusion gene. Use of a method of the invention is provided before, during and after treatment of a disease to evaluate the effectiveness of said treatment.

### EXAMPLE

### Detection of BCR-ABL fusion protein in precleared cell lysates

This example illustrates how normal (i.e. native) ABL can be removed from a sample prior to detection in a catching/detection antibody assay to improve the sensitivity of detecting the BCR-ABL fusion protein in an MRD assay. Such a "pre-clear" step can be performed with an anti-ABL antibody against the N-terminal part of ABL. All normal ABL expressed in the cells will be cleared, and only the ABL fragment that is present in the BCR-ABL fusion protein will bind to the detection beads carrying anti-ABL catching antibody against the C-terminal part of ABL. BCR-ABL bound to the beads is then detected using an anti-BCR detection antibody conjugated to a fluorescent label.

### Materials

1) Leukemic cells obtained from a MRD patient cultured in medium
2) Phosphate-buffered saline (PBS) /2% foetal calf serum (FCS): add 2 ml FCS (heat-inactivated) to 100 ml PBS pH 7.8; Store at 4 °C.
3) Radioimmunoprecipitation (RIPA)-buffer (without sodium deoxycholate):
   Preparation of 50 ml of RIPA-buffer:
      50 mM Tris-HCl: use 2.5 ml of a 1 M stock Tris-HCl
      150 mM NaCl: use 1.5 ml of a 5 M stock NaCl
      1% NP40: use 0.5 ml of undiluted Nonidet P40 (BDH, #56009 2L)
      0.1% SDS: use 0.5 ml of a 10% stock SDS
      Add 45 ml milli-Q.
      Store at 4 °C
4) Protease inhibitor cocktail, Sigma, #P2714:
   Dissolve the powder in 2 ml milli-Q to prepare a 50x concentrated solution. Store at 4 °C.
5) Preclear beads: Polystyrene beads (>10 µm) coated with depleting antibody directed against the N-terminus of ABL.
6) Detection beads: Luminex, PolyScience, or Molecular Probe beads, coated with catching antibody directed against the C-terminus of ABL.
7) Detection antibody: either FITC- or biotin-conjugated antibody directed against the N-terminus of BCR.
8) Streptavidin (SA)-PE, Caltag laboratories, #SA1004-1

### Methods

### Preparation of cell lysates

1) Transfer the number of cells needed and exactly calculated to a fresh tube and wash cells twice with PBS. Minimally 1*10⁶ cells are typically required for the analysis of a fusion protein. However, more cells e.g. up to 50*10⁶ cells may be needed if the MRD level (and therefore the levels of aberrant fusion protein) is expected to be very low.
2) In the meantime, calculate the volume of RIPA-buffer needed to make cell lysates: 50 µl RIPA-buffer per 500.000 cells. Transfer the RIPA-buffer to a 15 ml tube and add 50x concentrated protease inhibitor cocktail (20 µl protease inhibitor cocktail per 1 ml RIPA-buffer).
3) Resuspend the cell pellets in the appropriate amount of RIPA-buffer (5 x 10⁶/ml) containing protease inhibitors and transfer the solutions to fresh eppendorf-tubes.
4) Incubate on ice for 30 minutes.
5) Centrifuge 10 minutes at 10.000 *x g*, 4 °C.
6) Transfer the supernatant (cell lysate) to a fresh eppendorf-tube.

### Preclear of cell lysate

7) Add 1x10⁶ preclear beads to 100 µl of cell lysate sample.
8) Incubate on ice for 30 minutes.
9) Spin at 10.000 *x g* for 3 minutes and transfer the supernatant to a new eppendorf tube.
10) Repeat step 9.

### Detection of fusion protein

11) Add to one test-tube:
   - 100 µl of precleared cell lysate sample
   - 10.000 catching beads in 10 µl PBS + 2% FCS
   - 1-2 µg of conjugated detection antibody in 25 µl PBS + 2% FCS
12) Incubate on ice for 30 minutes.
13) Wash the beads with 500 µl PBS/2% FCS and spin for 1 minute at 14.000 x g. Remove supernatant and repeat this step.

When a biotin-labeled detection antibody is used, continue with steps 14 - 18. When a FITC-labeled detection antibody is used, continue with steps 17 and 18.
14) Dilute SA-PE 200 times in PBS/2% FCS and resuspend the beads in 20 µl of the diluted SA-PE per tube.
15) Incubate for 10 minutes at room temperature.
16) Wash the beads with 500 µl PBS/2% FCS and spin for 1 minute at 14.000xg. Remove supernatant and repeat this step.
17) After the second wash, resuspend the beads in 100-150 µl PBS/2% FCS per tube and transfer to FACS-tubes.
18) Measure the amount of label on the beads with a flow cytometer. The ratio of the amount of label and the initial number of cells is a measure of MRD level.

## Claims

1. A method for detecting a fusion protein in a sample, said fusion protein comprising an amino-terminal fragment and a carboxy-terminal fragment which are each corresponding to a native protein, said method comprising:
- contacting said sample with at least one binding molecule specifically reactive with a part of the native protein that is not present in the fusion protein, under conditions that allow for the formation of a complex between said at least one binding molecule and said native protein;
- removing said complex from said sample to deplete said sample of said native protein; and
- detecting said fusion protein in said sample using at least one antibody directed against said fusion protein.

2. A method according to claim 1 wherein said at least one binding molecule is an antibody or a binding fragment functionally equivalent thereto.

3. A method according to claims 1 or 2 wherein said at least one binding molecule is conjugated to a solid particle, preferably a bead.

4. A method according to claim 3, wherein said complex is removed from said sample by centrifugation or by magnetic separation.

5. A method according to any one of claims 1 to 4, wherein said fusion protein is detected using a set of at least a first and a second antibody, each antibody capable of recognizing a binding site positioned at opposite sides of the fusion region of said fusion protein.

6. A method according to any one of claims 1 to 5, whereby at least one antibody is labelled with a fluorochrome.

7. A method according to any one of claims 1 to 6, whereby at least one antibody is coupled to a bead.

8. A method according to any one of claims 1 to 7, wherein said fusion protein is detected via flow cytometry.

9. A method according to any one of the preceding claims further comprising after the step of removing said complex from said sample, detecting said complex using at least one reagent capable of specifically binding to said complex and determining the binding of said at least one reagent to said complex as an indication of the amount of native protein depleted from said sample.

10. A method according to claim 9 wherein said native protein is an abundant or housekeeping protein.

11. A method according to any one of claims 1 to 10, wherein said fusion protein comprises an amino-terminal fragment and a carboxy-terminal fragment which are each corresponding to a different non-tumour-specific protein.

12. A method according to claim 11 wherein said fusion protein is a result of a Philadelphia chromosome aberration.

13. A method according to claim 12 whereby the amino-terminal fragment of said fusion protein corresponds to the ABL or BCR protein whereas the carboxy-terminal fragment of said fusion protein corresponds to the BCR or ABL protein, respectively.

14. A method according to claim 13 whereby said at least one binding molecule, preferably an antibody, is specifically reactive with a fragment of the ABL or BCR protein, but not with said fusion protein.

15. A diagnostic kit for simultaneously detecting at least a first and a second tumour-specific fusion protein in a sample,
said first and second fusion protein comprising an amino-terminal fragment and a carboxy-terminal fragment which are each corresponding to a native protein,
said kit comprising at least one binding molecule specifically reactive with a part of the native protein that is not present in the first fusion protein, and one binding molecule specifically reactive with a part of the native protein that is not present in the second fusion protein;
and furthermore comprising at least a first and a second antibody for detecting said first and second tumour-specific fusion protein.

16. Kit according to claim 15, wherein said binding molecules are immobilized onto the same bead.

17. Use of a diagnostic kit according to claim 15 or 16 for the *in vitro* diagnosis and / or classification of a disease.

## Patentansprüche

1. Verfahren zum Nachweisen eines Fusionsproteins in einer Probe, wobei das Fusionsprotein ein aminoterminales Fragment und ein carboxyterminales Fragment umfasst, die jeweils einem nativen Protein entsprechen, wobei das Verfahren umfasst:
- In-Kontakt-Bringen der Probe mit wenigstens einem Bindungsmolekül, das spezifisch gegenüber einem Teil des nativen Proteins reaktiv ist, das in dem Fusionsprotein nicht vorhanden ist, unter Bedingungen, die die Bildung eines Komplexes zwischen dem wenigstens einen Bindungsmolekül und dem nativen Protein ermöglichen;
- Entfernen des Komplexes aus der Probe, so dass die Probe in Bezug auf das native Protein abgereichert wird; und
- Nachweisen des Fusionsproteins in der Probe unter Verwendung wenigstens eines Antikörpers, der gegen das Fusionsprotein gerichtet ist.

2. Verfahren gemäß Anspruch 1, wobei das wenigstens eine Bindungsmolekül ein Antikörper oder ein zu einem solchen funktionell äquivalentes Bindungsfragment ist.

3. Verfahren gemäß Anspruch 1 oder 2, wobei das wenigstens eine Bindungsmolekül mit einem festen Teilchen, vorzugsweise einem Bead, konjugiert ist.

4. Verfahren gemäß Anspruch 3, wobei der Komplex durch Zentrifugation oder durch magnetische Trennung aus der Probe entfernt wird.

5. Verfahren gemäß einem der Ansprüche 1 bis 4, wobei das Fusionsprotein unter Verwendung einer Menge von wenigstens einem ersten und einem zweiten Antikörper nachgewiesen wird, wobei die Antikörper jeweils in der Lage sind, eine Bindungsstelle zu erkennen, die sich auf entgegengesetzten Seiten des Fusionsbereichs des Fusionsproteins befinden.

6. Verfahren gemäß einem der Ansprüche 1 bis 5, wobei wenigstens ein Antikörper mit einem Fluorochrom markiert ist.

7. Verfahren gemäß einem der Ansprüche 1 bis 6, wobei wenigstens ein Antikörper an ein Bead gekoppelt ist.

8. Verfahren gemäß einem der Ansprüche 1 bis 7, wobei das Fusionsprotein mittels Durchflusscytometrie nachgewiesen wird.

9. Verfahren gemäß einem der vorstehenden Ansprüche, das nach dem Schritt des Entfernens des Komplexes aus der Probe weiterhin das Nachweisen des Komplexes unter Verwendung wenigstens eines Reagens, das spezifisch an den Komplex binden kann, und das Bestimmen der Bindung des wenigstens einen Reagens an den Komplex als Hinweis auf die Menge des aus der Probe abgereicherten nativen Proteins umfasst.

10. Verfahren gemäß Anspruch 9, wobei das native Protein ein reichlich vorhandenes oder Haushaltsprotein ist.

11. Verfahren gemäß einem der Ansprüche 1 bis 10, wobei das Fusionsprotein ein aminoterminales Fragment und ein carboxyterminales Fragment umfasst, die jeweils einem anderen nichttumorspezifischen Protein entsprechen.

12. Verfahren gemäß Anspruch 11, wobei das Fusionsprotein ein Ergebnis einer Philadelphia-Chromosomen-Aberration ist.

13. Verfahren gemäß Anspruch 12, wobei das aminoterminale Fragment des Fusionsproteins dem ABL- oder BCR-Protein entspricht, während das carboxyterminale Fragment des Fusionsproteins dem BCR- oder ABL-Protein entspricht.

14. Verfahren gemäß Anspruch 13, wobei das wenigstens eine Bindungsmolekül, vorzugsweise ein Antikörper, spezifisch gegenüber einem Fragment des ABL- oder BCR-Proteins, aber nicht gegenüber dem Fusionsprotein reaktiv ist.

15. Diagnostischer Kit zum gleichzeitigen Nachweisen wenigstens eines ersten und eines zweiten tumorspezifischen Fusionsproteins in einer Probe;
wobei das erste und das zweite Fusionsprotein ein aminoterminales Fragment und ein carboxyterminales Fragment umfassen, die jeweils einem nativen Protein entsprechen;
wobei der Kit wenigstens ein Bindungsmolekül, das spezifisch gegenüber einem Teil des nativen Proteins reaktiv ist, das in dem ersten Fusionsprotein nicht vorhanden ist, und wenigstens ein Bindungsmolekül, das spezifisch gegenüber einem Teil des nativen Proteins reaktiv ist, das in dem zweiten Fusionsprotein nicht vorhanden ist, umfasst;
und der weiterhin wenigstens einen ersten und einen zweiten Antikörper zum Nachweisen des ersten und des zweiten tumorspezifischen Fusionsproteins umfasst.

16. Kit gemäß Anspruch 15, wobei die Bindungsmoleküle auf demselben Bead immobilisiert sind.

17. Verwendung eines diagnostischen Kits gemäß Anspruch 15 oder 16 für die in-vitro-Diagnose und/oder -Klassifizierung einer Krankheit.

## Revendications

1. Procédé de détection d'une protéine de fusion dans un échantillon, ladite protéine de fusion comprenant un fragment amino-terminal et un fragment carboxy-terminal qui correspondent chacun à une protéine native, ledit procédé comprenant les étapes de :
- mettre en contact ledit échantillon avec au moins une molécule de fixation qui réagit spécifiquement avec une partie de la protéine native qui n'est pas présente dans la protéine de fusion, dans des conditions qui permettent la formation d'un complexe entre au moins ladite molécule de fixation et ladite protéine native ;
- le fait d'ôter ledit complexe dudit échantillon pour appauvrir ledit échantillon de ladite protéine native ; et
- le fait de détecter ladite protéine de fusion dans ledit échantillon en utilisant au moins un anticorps dirigé contre ladite protéine de fusion.

2. Procédé selon la revendication 1, dans lequel au moins ladite molécule de fixation est un anticorps ou un fragment de fixation qui lui est équivalent au point de vue fonctionnel.

3. Procédé selon la revendication 1 ou 2, dans lequel au moins ladite molécule de fixation est conjuguée avec une particule solide, de préférence une bille.

4. Procédé selon la revendication 3, dans lequel ledit complexe est ôté dudit échantillon par centrifugation ou par séparation magnétique.

5. Procédé selon l'une quelconque des revendications 1 à 4, dans lequel ladite protéine de fusion est détectée en utilisant un ensemble d'au moins un premier et un second anticorps, chaque anticorps étant capable de reconnaître un site de fixation positionné à des endroits opposés de la région de fusion de ladite protéine de fusion.

6. Procédé selon l'une quelconque des revendications 1 à 5, dans lequel au moins un anticorps est marqué avec un fluorochrome.

7. Procédé selon l'une quelconque des revendications 1 à 6, dans lequel au moins un anticorps est couplé à une bille.

8. Procédé selon l'une quelconque des revendications 1 à 7, dans lequel ladite protéine de fusion est détectée par cytométrie de flux.

9. Procédé selon l'une quelconque des revendications précédentes, qui comprend en outre, après l'étape de retrait dudit complexe dudit échantillon, le fait de détecter ledit complexe en utilisant au moins un réactif capable de se fixer de façon spécifique audit complexe et de déterminer la fixation dudit au moins un réactif audit complexe comme une indication de la quantité de protéine native déplétée dudit échantillon.

10. Procédé selon la revendication 9, dans lequel ladite protéine native est une protéine abondante ou une protéine de ménage.

11. Procédé selon l'une quelconque des revendications 1 à 10, dans lequel ladite protéine de fusion comprend un fragment amino-terminal et un fragment carboxy-terminal qui correspondent chacun respectivement à une protéine spécifique non tumorale différente.

12. Procédé selon la revendication 11, dans lequel ladite protéine de fusion est le résultat d'une aberration du chromosome Philadelphie.

13. Procédé selon la revendication 12, dans lequel le fragment amino-terminal de ladite protéine de fusion correspond à la protéine ABL ou BCR, tandis que le fragment carboxy-terminal de ladite protéine de fusion correspond à la protéine BCR ou ABL, respectivement.

14. Procédé selon la revendication 13, dans lequel au moins ladite molécule de fixation, de préférence un anticorps, réagit de façon spécifique avec un fragment de la protéine ABL ou BCR, mais pas avec ladite protéine de fusion.

15. Trousse de diagnostic pour la détection simultanée d'au moins une première et une seconde protéine de fusion spécifique de tumeur dans un échantillon, lesdites première et seconde protéines de fusion comprenant un fragment amino-terminal et un fragment carboxy-terminal qui correspondent chacun à une protéine native,
ladite trousse comprenant au moins une molécule de fixation réagissant de façon spécifique avec une partie de la protéine native qui n'est pas présente dans la première protéine de fusion, et une molécule de fixation réagissant de façon spécifique avec une partie de la protéine native qui n'est pas présente dans la seconde protéine de fusion ;
et comprenant en outre au moins un premier et un second anticorps pour détecter lesdites première et seconde protéines de fusion spécifiques de tumeur.

16. Trousse selon la revendication 15, dans laquelle lesdites molécules de fixation sont immobilisées sur la même bille.

17. Utilisation d'une trousse de diagnostic selon la revendication 15 ou 16 pour le diagnostic *in vitro* et/ou la classification d'une maladie.
